# EUROPEAN PATENT SPECIFICATION

(11) **EP 2 587 262 B1**
(45) Date of publication and mention of the grant of the patent: **30.09.2015**
(21) Application number: 12189996.7
(22) Date of filing: 25.10.2012
(51) Int. Cl.: G01N 33/50, G01N 15/14

(54) **Detection method of activated neutrophils and apparatus therefor**
Erfassungsverfahren für aktivierte Neutrophile und Vorrichtung dafür
Procédé de détection de neutrophiles activés et appareil à cet effet

(30) Priority: 25.10.2011 JP 2011234176
(43) Date of publication of application: 01.05.2013
(73) Proprietor: SYSMEX CORPORATION, Kobe-shi, Hyogo 651-0073 (JP)
(72) Inventor: Kono, Mari, Kobe-shi, Hyogo 651-0073 (JP); Takagi, Yuri, Kobe-shi, Hyogo 651-0073 (JP)
(74) Representative: Paemen, Liesbet R.J.

(56) References cited:
- EP-A1- 2 280 278
- US-A1- 2009 023 129
- US-A1- 2009 162 830
- US-A1- 2010 129 855
- US-A1- 2011 027 788

## Description

### FIELD OF THE INVENTION

The present invention relates to a method of detecting activated neutrophils in a biotic sample.

### BACKGROUND

Normal leukocytes are normally classified into granulocytes composed of neutrophils, eosinophils and basophils, lymphocytes, and monocytes. Among these, neutrophils account for 50 to 60% of leukocytes and play an important role in biophylaxis against pathogens such as bacteria and fungi. For example, when foreign bodies invade into an organism by bacterial infection or the like, neutrophils are activated by a stimulus caused by an inflammatory reaction or the like of the organism and migrate to the site of infection to phagocytose foreign bodies. Foreign bodies phagocytosed by neutrophils are sterilized by various degrading enzymes and reactive oxygen species (ROS) as a strong toxic substance produced inside neutrophils.

On the other hand, neutrophils activated by the stimulus (hereinafter, also called "activated neutrophils") may damage normal tissues in the organism. For example, excessive ROS produced by activated neutrophils is known to damage self tissues of the organism by ischemia reperfusion injury or an inflammatory reaction such as autoimmune disease. ROS produced by activated neutrophils is also known to be involved in acute respiratory distress syndrome, ulcerative colitis, and lesion formation such as acute gastric mucus/mucosa lesion. The involvement of activated neutrophils is also known in transfusion-related acute lung injury.

Activated neutrophils are involved in various kinds of disease and disorder as described above and thus, extremely important information for diagnosis of disease, grasp of clinical condition, and therapeutic monitoring can be obtained not only by classifying and counting leukocytes, but also by detecting activated neutrophils in a blood test.

In recent years, the classification and counting of leukocytes in a specimen is performed by an automated blood cell counter applying the principle of flow cytometry in the field of clinical laboratory test. Hemocyte classifying reagents for measurement by such an apparatus are also commercially available. If a specimen is measured by the automated blood cell counter using the reagent, a signal of each detected hemocyte appears in a predetermined region on a scattergram.

Some methods of classifying and counting leukocytes by using the reagent as described above are known in the relevant technology. For example, Japanese Patent No. 4248017 discloses a method of classifying and counting both of abnormal leukocytes and normal leukocytes by using a reagent kit combining a stain solution specifically staining RNA and a hemolyzing agent containing a cationic surfactant and an nonionic surfactant. U.S. Patent Application No. 2009/0023129 and U.S. Patent Application No. 2011/0027788 disclose a method of classifying leukocytes into four or five classes and counting each class by using a reagent containing a stain solution containing predetermined fluorochrome and a hemolyzing agent containing a cationic surfactant and/or an nonionic surfactant. European patent publication No. 2280278 discloses a process for classifying and counting leukocytes by measuring information on side-scattered light intensity, information on forward-scattered light intensity and information on side fluorescence intensity of the leukocytes and counting the leukocytes in each group. However, none of these patent documents discloses detection of activated neutrophils.

The literature by J. Linssen and others (Cytometry Part B (Clinical Cytometry), 74B, 295-309 (2008) by J. Linssen and others) can be cited as an example of measuring a specimen that could contain activated neutrophils. J. Linssen and others report that when the whole blood activated by a stimulating substance is measured by the automated blood cell counter XE-2100 (manufactured by Sysmex Corporation) as a specimen, average side scattered light intensity (NEUT-X) and average fluorescence intensity (NEUT-Y) of a cluster classified as neutrophils in a leukocyte classifying/counting channel (DIFF-channel) decrease compared with NEUT-X and NEUT-Y of an inactivated specimen.

### SUMMARY OF THE INVENTION

The scope of the present invention is defined solely by the appended claims, and is not affected to any degree by the statements within this summary.

An object of the present invention is to provide a detection method and an apparatus of activated neutrophils capable of classifying and counting normal leukocytes and which enables detection of activated neutrophils.

The present inventors examined how signals change in measurements using an automated blood cell counter of neutrophils that are activated/stimulated and producing reactive oxygen species. As a result, surprisingly, in contrast to the report by J. Linssen and others described above, the present inventors found that neutrophils producing reactive oxygen species are present between a cluster containing neutrophils and a cluster containing eosinophils and further, fluorescence intensity thereof is equal to that of normal neutrophils or less and scattered light intensity thereof is higher than that of normal neutrophils, that is, scattered light intensity thereof is between that of normal neutrophils and that of eosinophils before completing the present invention.

(1) A first aspect of the present invention is a detection method of activated neutrophils, comprising:
   (a) preparing a measurement sample in which erythrocytes in a biotic sample are hemolyzed and nucleic acid of leukocytes is stained by a nucleic acid staining fluorochrome;
   (b) irradiating the prepared measurement sample with light and measuring scattered light intensity and fluorescence intensity generated from particles in the measurement sample;
   (c) classifying the leukocytes in the biotic sample into at least a cluster containing neutrophils and a cluster containing eosinophils based on the acquired scattered light intensity and fluorescence intensity; and
   (d) detecting particles of which the fluorescence intensity is equal to that of the cluster containing the neutrophils or less and whose scattered light intensity is between that of the cluster containing the neutrophils and that of the cluster containing the eosinophils as activated neutrophils.
(2) The method according to (1), wherein the cluster containing the neutrophils further contains basophils.
(3) The method according to (1) or (2), wherein the activated neutrophils have vacuolar degeneration generated in cytoplasm.
(4) The method according to any one of (1) to (3), wherein particles present between the cluster containing the neutrophils and the cluster containing the eosinophils are counted in the (d).
(5) The method according to (4), wherein if a value counted in the (d) is larger than a predetermined value, the particles are detected as the activated neutrophils, wherein the predetermined value is based on data accumulated from measurements of biotic samples containing activated neutrophils.
(6) The method according to any one of (1) to (5), wherein erythrocytes in the measurement sample are hemolyzed by a surfactant.
(7) The method according to (6), wherein the surfactant is at least one selected from nonionic surfactants and cationic surfactants.
(8) The method according to any one of (1) to (7), wherein the scattered light intensity is side scattered light intensity.

According to a detection method for activated neutrophils in the present invention, classification/counting of normal leukocytes in a biotic sample and also detection of activated neutrophils are enabled.

### BRIEF DESCRIPTION OF THE DRAWINGS

FIG. 1 is a scattergram when normal leukocytes are measured by a flow cytometer.
FIG. 2 is a view showing an appearance of a measuring apparatus suitable for performing the method according to the present invention.
FIG. 3 is a function block diagram of a measuring unit.
FIG. 4 is a schematic diagram of a detection unit.
FIG. 5 is a diagram showing the flow of measuring a measurement sample by the detection unit.
FIG. 6 is a function block diagram of a data processing unit.
FIG. 7 is a flow chart of processing by the data processing unit.
FIG. 8 is a diagram showing an example of a result display screen displayed by the data processing unit.
FIG. 9 is a diagram showing a modification of the result display screen displayed by the data processing unit.
FIG. 10A and FIG. 10B are scattergrams obtained from measurements of Example 1.
FIG. 11 is a microphotograph showing a result of Reference Example 1.
FIG. 12 is a microphotograph showing a result of Reference Example 2.
FIG. 13 is a scattergram obtained from measurements of Example 2.

### DETAILED DESCRIPTION OF THE PREFERRED EMBODIMENTS

The preferred embodiments of the present invention will be described hereinafter with reference to the drawings.

In the embodiments of the present invention, the biotic sample is not specifically limited insofar as the sample is a body fluid sample containing leukocytes and, for example, blood, bone marrow liquid, or urine extracted from mammals, preferably humans or a sample taken for apheresis may be cited. The biotic sample may also be a sample that may contain activated neutrophils.

The term "activated neutrophils" herein means neutrophils activated by a stimulus and is used as a term indicating hemocytes to be distinguished from normal (non-stimulated) neutrophils contained in normal leukocytes.

In the embodiments of the present invention, neutrophils producing reactive oxygen species (ROS) can be cited as activated neutrophils. In the relevant technology, superoxide, hydroxy radicals, hydrogen peroxide, and singlet oxygen are known as ROS produced by activated neutrophils.

It is known in activated neutrophils phagocytosing foreign bodies that foreign bodies enter phagocytotic vacuoles and then granules containing degrading enzymes and ROS are fused with the vacuoles to form phagolysosome and vacuolar degeneration is recognized by a morphologic observation in cytoplasm of activated neutrophils. Therefore, in the embodiments of the present invention, neutrophils in which vacuolar degeneration occurs in cytoplasm are also included in activated neutrophils.

### [Detection method of activated neutrophils]

The detection method of activated neutrophils according to the present invention (hereinafter, also referred to simply as "the detection method") will be described below.

In the detection method according to the present invention, first a measurement sample in which erythrocytes in a biotic sample are hemolyzed and nucleic acid of leukocytes is stained by fluorochrome having nucleic acid stain property is prepared (preparation process). More specifically, the biotic sample, fluorochrome to stain nucleic acid, and a hemolyzing agent containing a surfactant to hemolyze erythrocytes and to damage cell membranes of leukocytes to such an extent that the fluorochrome can pass through to prepare the measurement sample.

In this preparation process, erythrocytes contained in the biotic sample is swiftly hemolyzed by the action of the hemolyzing agent and also cell membranes of leukocytes are damaged to such an extent that fluorochrome can pass through. Then, the nucleic acid of leukocytes is stained by the fluorochrome after the damaged cell membranes of leukocytes being passed through by the fluorochrome. If activated neutrophils are present in the biotic sample, nucleic acid of activated neutrophils is also stained.

### [Fluorochrome]

The fluorochrome used by a detection method according to the present invention is not specifically limited insofar as the fluorochrome can stain nucleic acid and can appropriately be selected in accordance with the wavelength of light irradiated from a light source. Such fluorochromes include, for example, propidium iodide, ethidium bromide, ethidium-acridine heterodimer, ethidium diazide, ethidium homodimer-1, ethidium homodimer-2, ethidium monoazide, trimethylene bis [[3- [[4-[[(3-methylbenzothiazole-3-ium) -2-il] methylene]-1, 4-dihydroquinoline] 1-il] propyl] dimethyl aminium]·tetraiodide (TOTO-1), 4- [(3-methylbenzothiazole-2 (3H) - ylidene) methyl] - 1 - [3- (trimethylaminio) propyl] quinolinium·diiodide (TO-PRO-1), N, N, N', N'-tetramethyl-N, N'-bis [3- [4- [3- [(3-methylbenzothiazole-3-ium) -2-il] -2-propenylidene]-1, 4-dihydroquinoline-1-il] propyl]-1, 3-propane diaminium-tetraiodide (TOTO-3), or 2- [3- [[1- [3- (trimethylaminio) propyl]-1, 4-dihydroquinoline]-4-ylidene]-1-propenyl]-3-methylbenzothiazole-3-ium·diiodide (TO-PRO-3) and fluorochrome represented by Formula (I). Among these fluorochromes, the fluorochrome represented by Formula (I) is preferable.

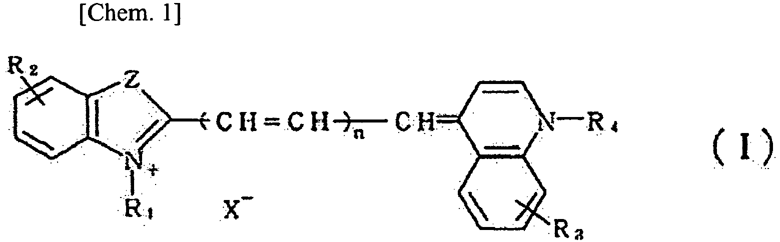

In the above Formula (I), R₁ and R₄ may be the same or different from each other and are an alkyl chain having a hydrogen atom, alkyl group, and hydroxy group, an alkyl chain having an ether group, an alkyl chain having an ester group, or a benzyl group that may have a substituent group; R₂ and R₃ may be the same or different from each other and are a hydrogen atom, hydroxyl group, halogen, alkyl group, alkenyl group, alkynyl group, alkoxy group, alkyl sulfonyl group, or phenyl group; Z is a sulfur atom, oxygen atom, or carbon atom having a methyl group; n is 0, 1, 2, or 3, and; X⁻ is an anion.

In the embodiments of the present invention, the alkyl group may be a straight-chain or branched chain alkyl group. If, in the embodiments of the present invention, one of R₁ and R₄ in the above Formula (I) is an alkyl group having 6 to 18 carbon atoms, the other is preferably a hydrogen atom or an alkyl group having less than six carbon atoms. Among alkyl groups having 6 to 18 carbon atoms, alkyl groups having 6, 8, or 10 carbon atoms are preferable.

In the embodiments of the present invention, for example, an alkyl group having 1 to 20 carbon atoms, an alkenyl group having 2 to 20 carbon atoms, and an alkynyl group having 2 to 20 carbon atoms can be cited as a substituent group of the benzyl group of R₁ and R₄ in the above Formula (I). Among these groups, the methyl group or ethyl group is particularly preferable.

In the embodiments of the present invention, for example, an alkenyl group having 2 to 20 carbon atoms can be cited as an alkenyl group of R₂ and R₃ in the above Formula (I). Also, an alkoxy group having 1 to 20 carbon atoms can be cited as an alkoxy group of R₂ and R₃. Among these groups, particularly the methoxy group or ethoxy group is preferable.

In the embodiments of the present invention, a halogen ion such as F⁻, Cl⁻, Br⁻, and I⁻, CF₃SO₃⁻, and BF₄⁻ can be cited as the anion X⁻ in the above Formula (I).

In the embodiments of the present invention, one fluorochrome or two or more fluorochromes may be used in the preparation process.

In the embodiments of the present invention, the fluorochrome to stain nucleic acid may be in a form of solution. The solvent to dissolve the fluorochrome is not specifically limited and, for example, water, an organic solvent, and a mixture of these can be cited. As the organic solvent, for example, alcohol, ethylene glycol, and dimethyl sulfoxide (DMSO) can be cited. The fluorochrome is preferably dissolved in an organic solvent because preservation stability in an aqueous solution may be undermined.

When a solution of the fluorochrome is used in the preparation process, the concentration of the fluorochrome can properly be set and is normally 0.01 to 100 µg/L, preferably 0.1 to 10 µg/L. If, for example, the fluorochrome represented by the above Formula (I) is used as the fluorochrome, the concentration of the fluorochrome in the solution is preferably 0.2 to 0.6 µg/L, more preferably 0.3 to 0.5 µg/L.

In the embodiments of the present invention, a stain reagent for leukocyte measurement available on the market may be used as the fluorochrome. As such a stain reagent, for example, Stromatolyser-4DS (Sysmex Corporation) can be cited. Stromatolyser-4DS is a stain reagent containing the fluorochrome represented by the above Formula (1).

### [Hemolyzing agent]

The hemolyzing agent used by the detection method according to the present invention contains a surfactant to hemolyze erythrocytes and to damage cell membranes of leukocytes to such an extent that the fluorochrome can pass through. Such a surfactant includes a cationic surfactant and an nonionic surfactant.

In the embodiments of the present invention, quarternary ammonium salt surfactant and pyridinium salt surfactant are cited as the cationic surfactant. As the quarternary ammonium salt surfactant, for example, a surfactant represented by Formula (II) shown below and having 9 to 30 carbon atoms in all is suitably used.

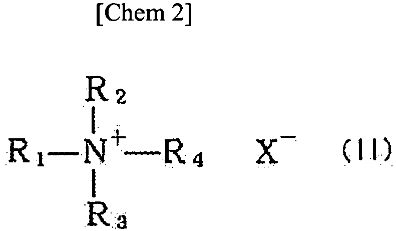

In the above Formula (II), R₁ is an alkyl group or an alkenyl group having 6 to 18 carbon atoms; R₂ and R₃ may be the same or different from each other and are an alkyl group or an alkenyl group having 1 to 4 carbon atoms; R₄ is an alkyl group or an alkenyl group having 1 to 4 carbon atoms or a benzyl group; and X⁻ is a halogen atom.

In the above Formula (II), an alkyl group or an alkenyl group having 6, 8, 10, 12, or 14 carbon atoms is preferable as R₁ and particularly preferable is a straight-chain alkyl group. As more concrete R₁, the octyl group, decyl group, and dodecyl group can be cited. As R₂ and R₃, the methyl group, ethyl group, or propyl group is preferable. As R₄, the methyl group, ethyl group, or propyl group is preferable.

As the pyridinium salt surfactant, for example, a surfactant represented by Formula (III) shown below is suitably used.

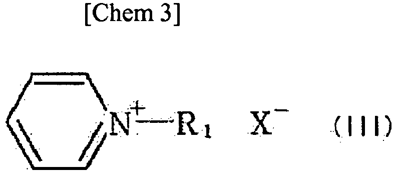

In the above Formula (III), R₁ is an alkyl group or an alkenyl group having 6 to 18 carbon atoms and
X⁻ is a halogen atom.

In the above Formula (III), an alkyl group or an alkenyl group having 6, 8, 10, 12, or 14 carbon atoms is preferable as R₁ and particularly preferable is a straight-chain alkyl group. As more concrete R₁, the octyl group, decyl group, and dodecyl group can be cited.

In the embodiments of the present invention, a polyoxyethylene nonionic surfactant represented by Formula (VI) shown below is suitably used as the nonionic surfactant.

[Chem 4] R₁-R₂-(CH₂CH₂O)ₙ-H (VI)

In the above Formula (VI), R₁ is an alkyl group, an alkenyl group, or an alkynyl group having 8 to 25 carbon atoms, R₂ is -O-, -COO-, or 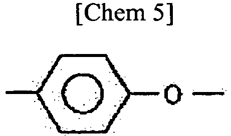 , and n is an integer of 10 to 50.

As concrete examples of the above nonionic surfactant, polyoxyethylene alkyl ether, polyoxyethylene sterol, polyoxyethylene castor oil, polyoxyethylene sorbitol fatty acid ester, polyoxyethylene alkylamine, and polyoxyethylene polyoxypropylene alkyl ether can be cited.

In the embodiments of the present invention, one surfactant or two or more surfactants may be contained in the hemolyzing agent. When two surfactants or more are contained, cationic surfactant and nonionic surfactant may be combined, only cationic surfactants may be combined, or only nonionic surfactants may be combined.

In the embodiments of the present invention, the surfactant may be in a form of solution. The solvent to dissolve the surfactant is not specifically limited and, for example, water, an organic solvent, and a mixture of these can be cited. As the organic solvent, for example, alcohol, ethylene glycol, and DMSO can be cited.

When a solution of the surfactant is used in the preparation process, the concentration thereof can properly be set in accordance with the type of the surfactant and is normally 10 to 10000 ppm, preferably 100 to 1000 ppm for a cationic surfactant and 10 to 100000 ppm, preferably 100 to 10000 ppm, and particularly preferably 1000 to 5000 ppm for an nonionic surfactant.

In the embodiments of the present invention, the hemolyzing agent may contain aromatic organic acid. As the aromatic organic acid, for example, phthalic acid, benzoic acid, salicylic acid, hippuric acid, p-aminobenzenesulfonic acid, benzenesulfonic acid, and salts thereof are suitably used. One aromatic organic acid or two or more aromatic organic acids may be contained in the hemolyzing agent.

In the embodiments of the present invention, the hemolyzing agent may contain a buffer to maintain pH constant. As such a buffer, for example, citrate, HEPES, and phosphate can be cited. When the aromatic organic acid displays a buffer action, the addition of a buffer to the hemolyzing agent is optional.

In the embodiments of the present invention, pH and the osmotic pressure of the hemolyzing agent are not specifically limited, but pH is preferably 5.0 to 9.0 and the osmotic pressure is preferably 20 to 150 mOsm/kg in terms of efficient hemolysis of erythrocytes. pH of the hemolyzing agent can be adjusted by adding a pH regulator such as sodium hydroxide and hydrochloric acid. The osmotic pressure of the hemolyzing agent can be prepared by adding an osmoregulating chemical such as sugar, amino acid, an organic solvent, and sodium chloride. As the sugar, for example, glucose, xylitol, mannitol, arabinose, and ribitol can be cited. As the amino acid, alanine, proline, glycine, and valine can be cited. As the organic solvent, ethylene glycol and glycerin can be cited.

In the embodiments of the present invention, a commercially available reagent for leukocytes measurement may be used as the hemolyzing agent. As such a reagent, for example, Stromatolyser-4DL (Sysmex Corporation) can be cited. Stromatolyser-4DL is a hemolyzing agent containing the cationic surfactant, nonionic surfactant, and aromatic organic acid described above and whose pH and osmotic pressure are within the above ranges.

### [Preparation of a measurement sample]

In the above preparation process, the order of mixing the biotic sample, fluorochrome, and hemolyzing agent is not specifically limited. For example, the fluorochrome and hemolyzing agent may first be mixed before a mixed solution thereof and the biotic sample being mixed. Alternatively, the hemolyzing agent and biotic sample may first be mixed before a mixed solution thereof and the fluorochrome being mixed. In the embodiments of the present invention, the same results can be obtained by mixing in any order.

In the embodiments of the present invention, the biotic sample, fluorochrome, and hemolyzing agent are mixed so that the volume ratio of the biotic sample: fluorochrome + hemolyzing agent becomes 1:5 to 1:1000, preferably 1:10 to 1:100. In this case, the ratio of the fluorochrome to the hemolyzing agent in the mixture is 1:1 to 1:1000, preferably 1:10 to 1:100. By mixing the biotic sample, fluorochrome, and hemolyzing agent in such a ratio, hemolysis of erythrocytes proceeds swiftly and nucleic acid of leukocytes can be stained. The sufficient amount of biotic sample is about 5 to 500 µL.

In the embodiments of the present invention, after the biotic sample, fluorochrome, and hemolyzing agent being mixed, it is preferable to incubate the mixture at 15°C to 50°C, preferably 30°C to 45°C for 5 to 120 seconds, preferably 5 to 30 seconds.

### [Measurements of scattered light intensity and fluorescence intensity]

According to a method of the present invention, the measurement sample prepared in the above process is irradiated with light to measure scattered light intensity and fluorescence intensity generated from particles in the sample (measurement process).

In the embodiments of the present invention, it is preferable to perform the measurement process by using a flow cytometer. In measurements by using the flow cytometer, scattered light intensity and fluorescence intensity of a signal emitted from particles, for example, stained leukocytes in the sample by the measurement sample being irradiated with light when passing through flow cells of the flow cytometer can be obtained.

In the embodiments of the present invention, the scattered light intensity is not specifically limited insofar as the intensity of scattered light can be measured by a commercially available flow cytometer and, for example, intensity of forward scattered light (for example, around the light-receiving angle 0 to 20 degrees) and side scattered light (around the light-receiving angle 90 degrees) can be cited.

In the relevant technology, side scattered light is known to reflect internal information about cell nuclei and granules and forward scattered light is known to reflect information about the size of cells. In the embodiments of the present invention, it is preferable to measure side scattered light intensity as the scattered light intensity.

The fluorescence intensity is information obtained by measuring excited florescence emitted from nucleic acid or the like inside cells stained by the fluorochrome when the measurement sample is irradiated with excitation light of an appropriate wavelength. The wavelength of excitation light and that of received light can appropriately be selected in accordance with the type of the fluorochrome used.

In the embodiments of the present invention, the light source of the flow cytometer is not specifically limited and the light source of a wavelength suitable for exciting the fluorochrome may be selected. As such a light source, for example, a red semiconductor laser, blue semiconductor laser, argon laser, He-Ne laser, mercury arc lamp or the like is used. Particularly, the semiconductor laser is inexpensive compared with a gas laser and thus is preferable.

### [Classification of leukocytes and detection of activated neutrophils]

According to a method of the present invention, leukocytes in the above biotic sample are classified into at least a cluster containing neutrophils and a cluster containing eosinophils based on the acquired scattered light intensity and fluorescence intensity (classification process) and activated neutrophils are detected (detection process).

In the classification process, if a measured particle is a normal leukocyte, the particle is classified into one of four clusters of lymphocytes, monocytes, eosinophils, and granulocytes other than eosinophils based on scattered light intensity and fluorescence intensity. Granulocytes other than eosinophils represent neutrophils and basophils. Because the number of basophils contained in a cluster of neutrophils and basophils is very small, a cluster of neutrophils and basophils can be considered as a cluster of neutrophils. Then, in the detection process, among measured particles, particles present between a cluster containing neutrophils and a cluster containing eosinophils are detected as activated neutrophils. Preferably, among measured particles, particles whose fluorescence intensity is equal to that of the cluster containing neutrophils or less and whose scattered light intensity is between that of the cluster containing neutrophils and that of the cluster containing eosinophils are detected as activated neutrophils.

In the embodiments of the present invention, normal leukocytes may be classified into five clusters of lymphocytes, monocytes, neutrophils, eosinophils, and basophils.

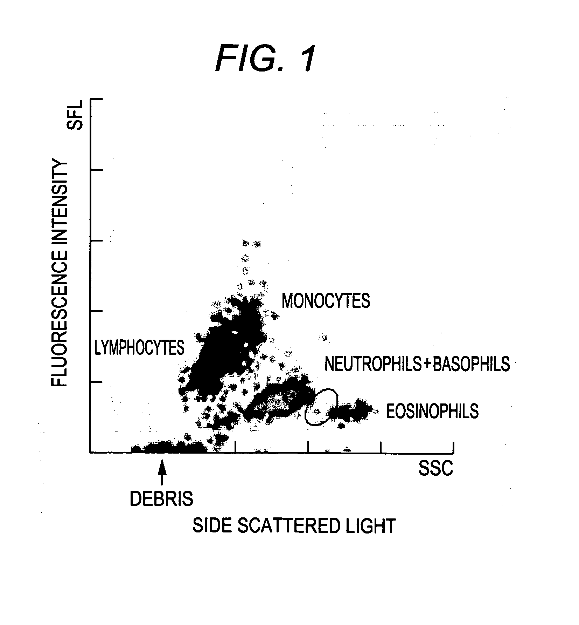

In a preferred embodiment of the present invention, leukocytes are classified based on a scattergram created and obtained by setting two axes of scattered light intensity and fluorescence intensity. In the relevant technology, regions where each cluster of lymphocytes, monocytes, neutrophils, eosinophils, and basophils appear on the scattergram are determined in advance from data accumulated for normal leukocytes. Therefore, particles as normal leukocytes are classified, as shown in FIG. 1, into four clusters by creating a scattergram in which, for example, the side scattered light intensity is set as the horizontal axis and the fluorescence intensity is set as the vertical axis. Further, by using appropriate analysis software, a window surrounding each cluster on the scattergram can be provided to count the number of cells therein to acquire the number of cells of each cluster.

In the above detection of activated neutrophils, if at least a particle is present between a cluster containing neutrophils and a cluster containing eosinophils, preferably at least a particle (a particle surrounded by a round frame in FIG. 1) whose fluorescence intensity is equal to that of the cluster containing neutrophils or less and whose scattered light intensity is between that of the cluster containing neutrophils and that of the cluster containing eosinophils is present, the particle may be detected as an activated neutrophil.

In another embodiment of the present invention, activated neutrophils may be detected as described below. First, particles present between a cluster containing neutrophils and a cluster containing eosinophils, preferably particles whose fluorescence intensity is equal to that of the cluster containing neutrophils or less and whose scattered light intensity is between that of the cluster containing neutrophils and that of the cluster containing eosinophils are detected and counted. Next, if the counted number of particles is larger than a predetermined value, the particles are detected as activated neutrophils. The predetermined value can appropriately be set based on data accumulated from measurements of biotic samples containing activated neutrophils.

### [Measuring apparatus]

A measuring apparatus suitable for performing a method according to the present invention is an apparatus to classify leukocytes in a biotic sample and also to detect activated neutrophils.

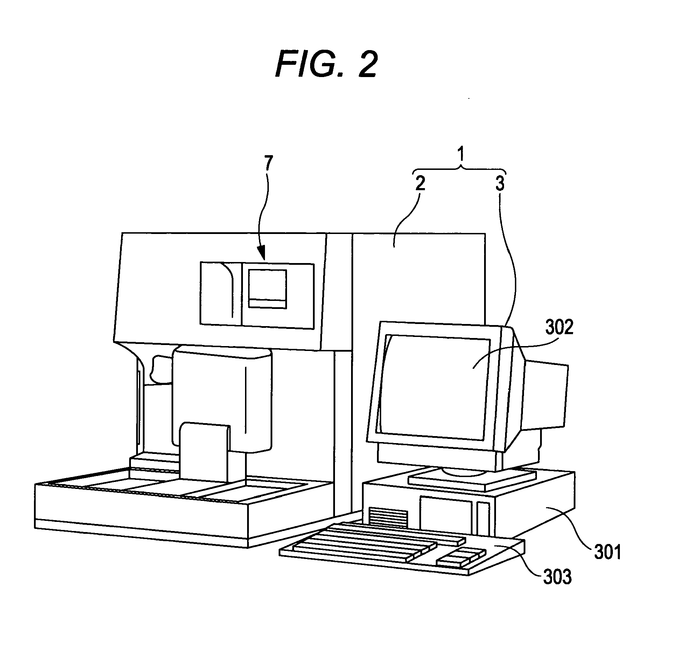

FIG. 2 shows the appearance of an embodiment of the measuring apparatus suitable for performing the method according to the present invention. A measuring apparatus 1 suitable for performing the method according to the present embodiment is configured as an automated multi-item blood cell analyzer and includes a measuring unit 2 and a data processing unit 3. The measuring unit 2 includes an apparatus that mixes a biotic sample and the above fluorochrome and hemolyzing agent to prepare a measurement sample and an apparatus that measures scattered light intensity and fluorescence intensity of the obtained measurement sample. The data processing unit 3 includes an apparatus that analyzes a measurement result output from the measuring unit 2 to classify leukocytes and to detect activated neutrophils.

In FIG. 2, the measuring unit 2 and the data processing unit 3 are configured as separate units, but both units may be configured as an integrated unit.

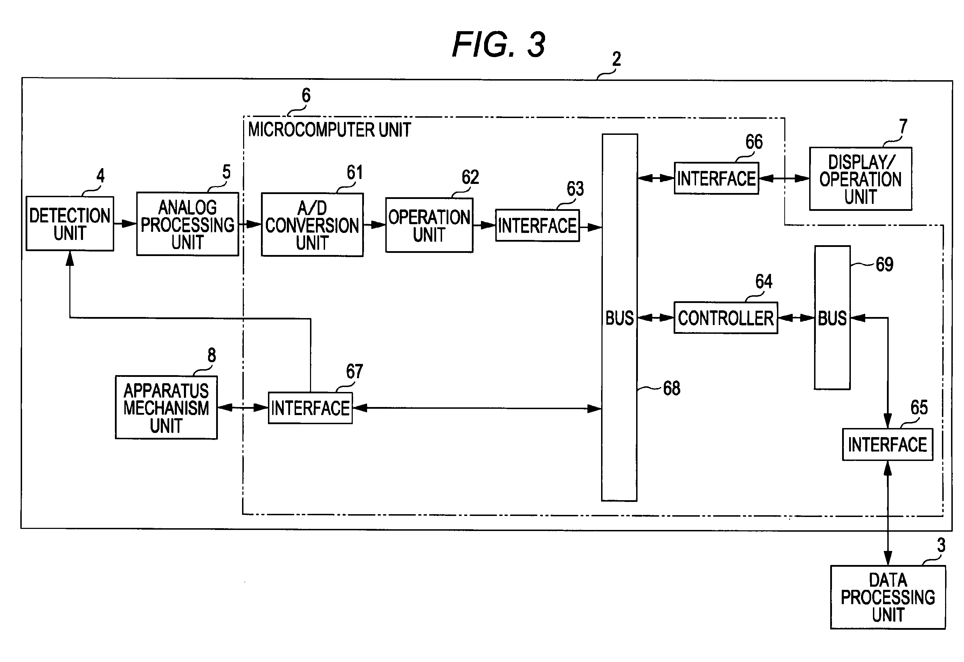

FIG. 3 shows a block diagram of the measuring unit 2. As shown in FIG. 2, the measuring unit 2 includes a detection unit 4 of hemocytes, an analog processing unit 5 of output from the detection unit 4, a microcomputer unit 6, a display/operation unit 7, and an apparatus mechanism unit 8.

The detection unit 4 includes a leukocyte detection unit that detects leukocytes. The detection unit 4 also includes, in addition to the leukocyte detection unit, an RBC/PLT detection unit that measures the number of erythrocytes and the number of platelets, an HGB detection unit that measures the amount of hemoglobin in blood, and an IMI detection unit that detects juvenile particles. The detection unit 4 is configured as an optical detection unit, more specifically, as a detection unit by the flow cytometry.

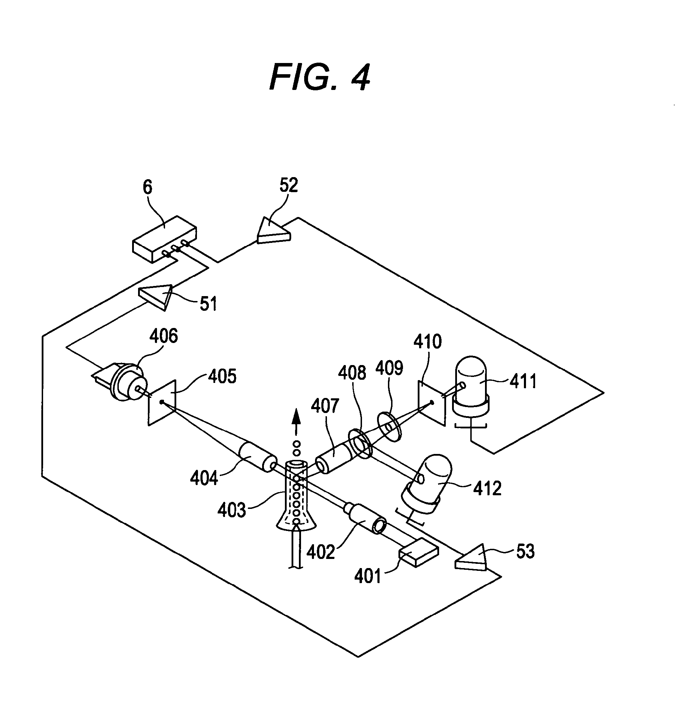

FIG. 4 shows the configuration of an optical system of the detection unit 4. In FIG. 4, a beam emitted from a laser diode 401 is irradiated on particles (such as leukocytes) passing through a sheath flow cell 403 via an irradiation lens system 402. The detection unit 4 detects forward scattered light, side scattered light, and side fluorescence emitted from particles inside the sheath flow cell irradiated with light.

As shown in FIG. 4, forward scattered light emitted from particles (such as leukocytes) passing through the sheath flow cell 403 is received by a photodiode (forward scattered light receiving unit) 406 via a condenser lens 404 and a pinhole unit 405. Side scattered light is received by a photo multiplier (side scattered light receiving unit) 411 via a condenser lens 407, a dichroic mirror 408, an optical filter 409, and a pinhole unit 410. Side fluorescence is received by a photo multiplier (side fluorescence receiving unit) 412 via the condenser lens 407 and the dichroic mirror 408.

Analog processing such as amplification/waveform processing is performed by the analog processing unit 5 having amplifiers 51, 52, 53 respectively, on received signals output from the light receiving units 406, 411, and 412 before the received signals being sent to the microcomputer unit 6.

As shown in FIG. 3, the microcomputer unit 6 includes an A/D conversion unit 61, an operation unit 62 that performs predetermined operation processing on a digital signal output from the A/D conversion unit 61, a control processor, and a controller 64 constituted of a memory for a control processor operation. Further, the microcomputer unit 6 includes an interface unit 66 interposed between the microcomputer unit 6 and the display/operation unit 7 and an interface unit 67 interposed between the microcomputer unit 6 and the apparatus mechanism unit 8.

The operation unit 62 is connected to the controller 64 via an interface unit 63 and a bus 68. The controller 64 is also connected to the detection unit 4 and the apparatus mechanism unit 8 via the interface units 66, 67 and the bus 68 and to the data processing unit 3 via an interface unit 65 and a bus 69.

The A/D conversion unit 61 converts a received signal output from the analog processing unit 5 into a digital signal and outputs the digital signal to the operation unit 62. The operation unit 62 performs predetermined operation processing on a digital signal output from the A/D conversion unit 61. Then, the operation unit 62 outputs an operation result (measurement result) to the controller 64 via the interface unit 63 and the bus 68.

The controller 64 is connected to the data processing unit 3 via the external interface unit 65 and can send an operation result output from the operation unit 62 to the data processing unit 3. The controller 64 also exercises control of a sampler (not shown) that automatically supplies a sample container and the apparatus mechanism unit 8 constituted of a fluid system and the like for preparation/measurement of samples and other control.

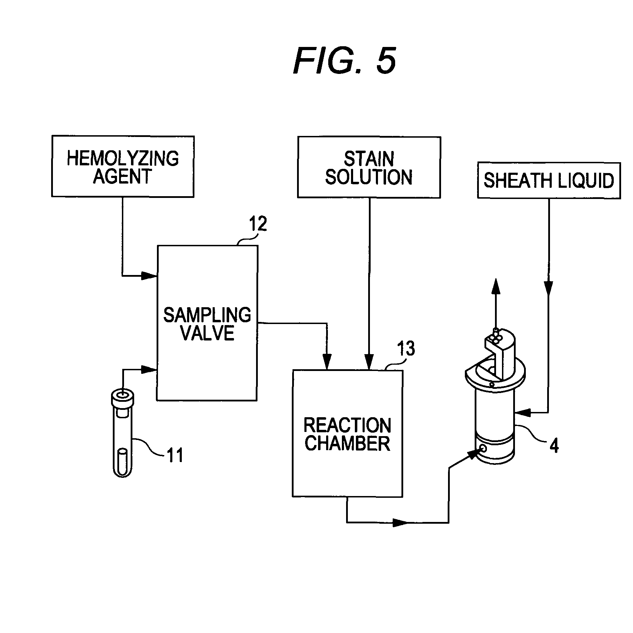

FIG. 5 shows a state in which a biotic sample, fluorochrome, and hemolyzing agent are mixed in the measuring unit 2 to prepare a measurement sample and the obtained measurement sample is measured by the detection unit 4.

In FIG. 5, the biotic sample inside a sample container 11 is sucked from a suction pipette (not shown) into a sampling valve 12. The biotic sample quantified by the sampling valve 12 is mixed with a predetermined amount of hemolyzing agent and the obtained mixture is carried into a reaction chamber 13. A predetermined amount of fluorochrome is supplied into the reaction chamber 13 to be mixed with the mixture. Erythrocytes in the biotic sample are hemolyzed by allowing the mixture of the biotic sample, fluorochrome, and hemolyzing agent to react in the reaction chamber 13 for a predetermined time to obtain a measurement sample in which leukocytes are stained.

The obtained measurement sample is sent to the detection unit 4 together with a sheath liquid (for example, Cell-Pack (II) manufactured by Sysmex Corporation) and measured by the detection unit 4 according to the flow cytometry.

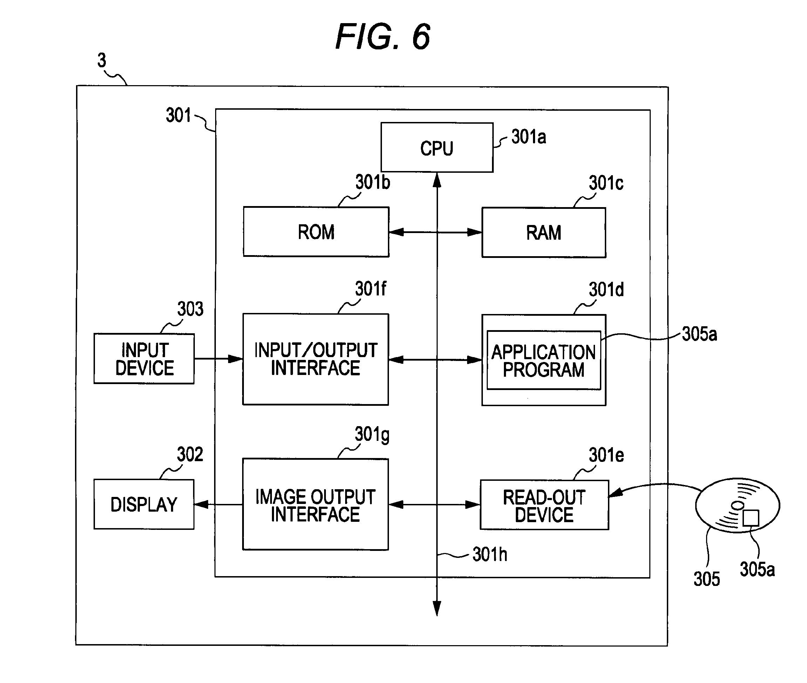

FIG. 6 shows the configuration of the data processing unit 3. The data processing unit 3 mainly includes a body 301, a display unit 302, and an input device 303. In the body 301, a CPU 301a, a ROM 301b, a RAM 301c, a hard disc 301d, a read-out device 301e, an input/output interface 301f, and an image output interface 301g are connected so that data can mutually be communicated by a bus 301h.

The CPU 301a can execute a computer program stored in the ROM 301b and a computer program loaded into the RAM 301c. Each function block described later is realized by an application program 305a described later being executed by the CPU 301a and a computer functions as the data processing unit 3.

The ROM 301b is configured by mask ROM, PROM, EPROM, EEPROM, and the like, and is recorded with the computer program to be executed by the CPU 301a and data used for the same.

The RAM 301c is configured by SRAM, DRAM, or the like. The RAM 301c is used to read out the computer program recorded on the ROM 301b and the hard disc 301d. The RAM 301c is also used as a work area when the CPU 301a executes the computer programs.

The hard disc 301 d is installed with various computer programs for the CPU 301a to execute such as operating system and application programs, and data used in execution of the computer programs. The application program 305a (computer program for detection of activated neutrophils) described later and predetermined values used for detection of activated neutrophils are also installed on the hard disc 301d.

The read-out device 301e is configured by flexible disc drive, CD-ROM drive, DVD-ROM drive, or the like. The read-out device 301e can read out a computer program or data recorded on a portable recording medium 305. The application program 305a for a computer to execute an operation is stored on the portable recording medium 305. The CPU 301a can also read out the application program 305a from the portable recording medium 305 and install the application program 305a on the hard disc 301d.

The application program 305a is not only provided by the portable recording medium 305, but can also be provided through communication line (wired or wireless) from external devices capable of communicating and connected to the data processing unit 3 through the communication line. For instance, the application program 305a may be stored in the hard disc of a server computer on the Internet, so that the data processing unit 3 can access the server computer to download the computer program and install the computer program on the hard disc 301d.

The operating system that provides the graphical user interface environment such as Windows (registered trademark) manufactured and sold by US Microsoft Corporation is installed on the hard disc 301d. In the following description, the application program 305a suitable for performing the method according to an embodiment of the present invention is assumed to operate on the operating system.

The input/output interface 301f is configured by a serial interface such as USB, IEEE1394, RS-232C, a parallel interface such as SCSI, IDE, IEEE1284, and an analog interface configured by a D/A converter, A/D converter, and the like. An input device 303 including a keyboard and mouse is connected to the input/output interface 301f. Thus, data can be input into the computer body 301 by the input device 303 being used by the user.

The image output interface 301g is connected to the display unit 302 configured by LCD, CRT, or the like and outputs a video signal in accordance with image data provided from the CPU 301a to the display unit 302. The display unit 302 displays the image data (screen) according to the input video signal.

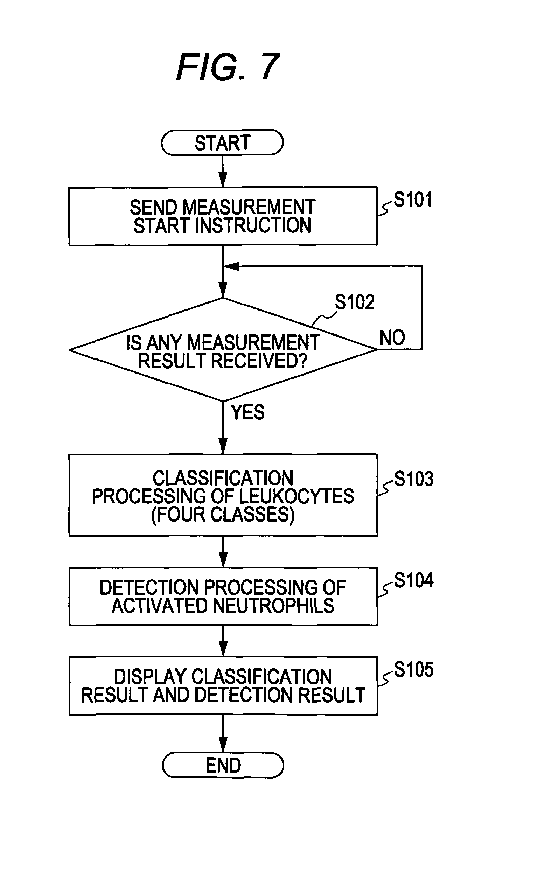

FIG. 7 shows a processing flow of the data processing unit 3 based on a computer program suitable for detecting activated neutrophils according to an embodiment of the method of the present invention.

First, a measurement start instruction is sent from the data processing unit 3 to the measuring unit 2 by a user's operation or the like (step S101). The CPU 301a of the data processing unit 3 waits until a measurement result is received from the measuring unit 2 (NO in step S102).

In response to the received measurement start instruction, the measuring unit 2 prepares and measures a measurement sample and sends a measurement result to the data processing unit 3. When the measurement result is received from the measuring unit 2 via the input/output interface 301f (YES in step S102), the CPU 301 a of the data processing unit 3 causes the RAM 301c to store the received measurement result and performs classification processing of leukocytes on the measurement result (step S103).

In a preferred embodiment of the program disclosed herein, in step S103, the data processing unit 3 creates scattergram (particle distribution map) in which the side scattered light intensity is set as the horizontal axis and the fluorescence intensity is set as the vertical axis. The CPU 301a of the data processing unit 3 reads out data on regions where each cluster of lymphocytes, monocytes, neutrophils, eosinophils, and basophils is predicted to appear on the scattergram the hard disc 301d is caused to store in advance to analyze the created scattergram based on the data. Accordingly, each cluster on the scattergram is classified into four kinds of clusters of "particle cluster of lymphocytes", "particle cluster of monocytes", "particle cluster of neutrophils and basophils", and "particle cluster of eosinophils" to recognize each cluster. In this step, the data processing unit 3 may count particles in each cluster by the analysis.

Based on the classification result in step S103, if any particle is present between a cluster containing neutrophils and a cluster containing eosinophils, preferably if any particle whose fluorescence intensity is equal to that of the cluster containing neutrophils or less and whose scattered light intensity is between that of the cluster containing neutrophils and that of the cluster containing eosinophils is present, the data processing unit 3 detects and counts relevant particles and recognizes such particles as activated neutrophils (step S104). In this case, "particle cluster of neutrophils and basophils" is considered as "particle cluster of neutrophils".

In an embodiment of the program disclosed herein, in step S104, the CPU 301a of the data processing unit 3 may read out data on the predetermined value used for detection of activated neutrophils the hard disc 301d is caused to store in advance, so that the presence of activated neutrophils in the sample is recognized if the number of detected particles whose fluorescence intensity is lower than that of neutrophils and whose scattered light intensity is between that of neutrophils and that of eosinophils is larger than the predetermined value.

In another embodiment of the program disclosed herein, in step S104, if at least one particle present between a cluster containing neutrophils and a cluster containing eosinophils is detected, preferably if at least one particle whose fluorescence intensity is equal to that of the cluster containing neutrophils or less and whose scattered light intensity is between that of the cluster containing neutrophils and that of the cluster containing eosinophils is detected, the CPU 301a of the data processing unit 3 may recognize the presence of activated neutrophils in the sample.

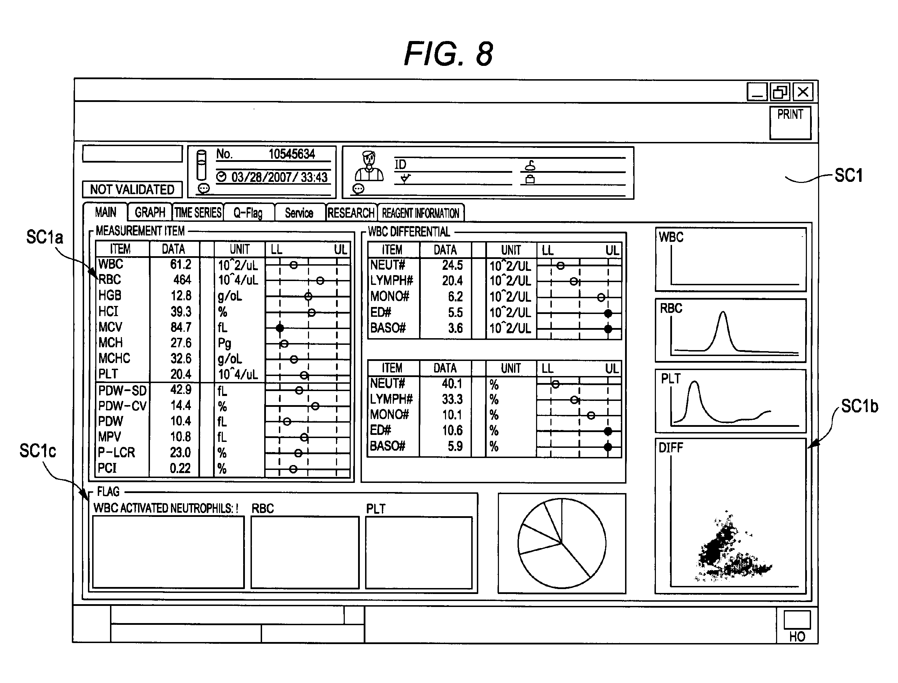

The CPU 301a of the data processing unit 3 causes the hard disc 301d to store the above classification result and detection result and also outputs the results to the display unit 302 via the image output interface 301g (step S105). FIG. 8 shows an example of a result display screen SC1 displayed in the display unit 302 by the data processing unit 3. The result display screen SC1 contains a display area SC1a to display numerical data of each measurement item and a display area SC1b to display a scattergram showing a distribution of numbers and sizes of particles of predetermined items (such as WBC, RBC, PLT, and DIFF).

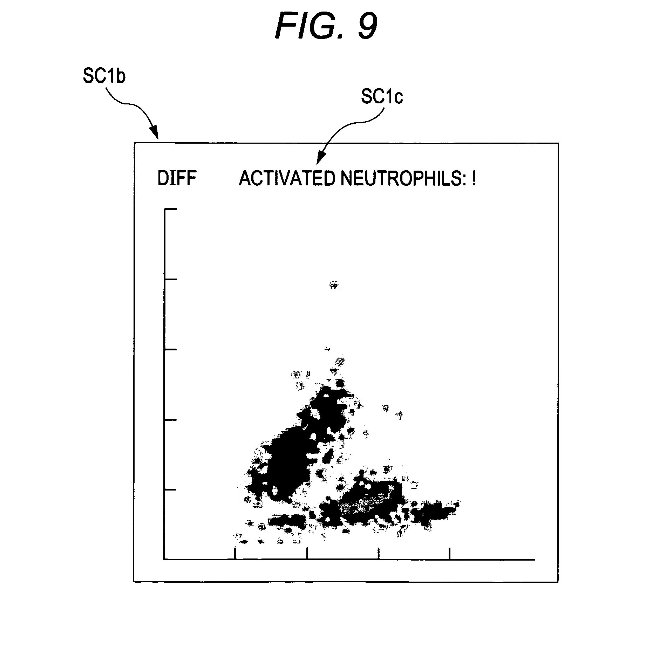

If, in step S104, any activated neutrophil is detected, a note SC1c is displayed in the result display screen SC1. The note SC1c indicates that activated neutrophils are present in the biotic sample. FIG. 9 shows an example displaying the note SC1c in the display area SC1b.

Hereinafter, the method of the present invention will be described in detail with reference to examples, however, the present invention is not limited to the examples.

### [Examples]

### (Example 1)

Peripheral blood is extracted from an able-bodied person by using a heparin blood collection tube for hematologic examination. A polynuclear sample of purity 90% or more is obtained by centrifugation of the obtained blood using a monopolymer separation solution (Dainippon Sumitomo Pharma Co., Ltd.) at room temperature. This operation is performed according to instructions of the manufacturer (Dainippon Sumitomo Pharma Co., Ltd.). Then, the obtained polymorphonucleocytes (2 X 10⁵) are suspended in a 1 mL of 0.1% bovine serum albumin (phosphate buffered saline). To stain neutrophils of polymorphonucleocytes, CD16 (antihuman CD16 Fc gamma receptor III (clone: DJ130c)·mouse monoclonal antibody/neutrophil specific antibody for RPE labeled flow cytometry; Dako) (final concentration: 750 µg/L) fluorescencelabeled by phycoerythrin (PE) is added to the obtained suspension for incubation at room temperature for 10 min. Next, APF (Aminophenyl Fluorescein, Sekisui Medical Co., Ltd.) (final concentration: 10 µM) as a reagent for ROS detection is added to the suspension, which is left still at room temperature for 30 min. Lastly, PMA (Phorbol 12-myristate 13-acetate, Sigma-Aldrich Co., LLC.) (final concentration: 0.32 nM) or fMLP (N-Formyl-Met-Leu-Phe, Sigma-Aldrich Co., LLC.) (final concentration: 50 nM) as a stimulating substance to activate neutrophils is added to the suspension, which is left still at room temperature for 10 min to prepare a reagent containing activated neutrophils.

Also as a control sample, a sample treated in the same manner as above except that neither PMA nor fMLP is added is prepared.

The above sample of 9 µL, Stromatolyser-4DL (442 µL) and Stromatolyser-4DS (9 µL) (both are manufactured by Sysmex Corporation) are mixed and incubated at room temperature for 2 min to obtain a measurement sample. Stromatolyser-4DL is a diluent containing a surfactant that hemolyzes erythrocytes and damages cell membranes of leukocytes to such an extent that the fluorochrome can pass through. Stromatolyser-4DS is a solution containing a nucleic acid staining fluorochrome.

The measurement sample is measured by the general-purpose flow cytometry FACSCalibur (manufactured by Becton, Dickinson and Company) to obtain scattergrams of "CD16 derived fluorescence intensity (CD16-PE, 585 nm)-forward scattered light intensity (FSC)", "nucleic acid staining fluorochrome derived fluorescence intensity in Stromatolyser-4DS (4DS, 665 nm)-side scattered light intensity (SSC)", and "APF derived fluorescence intensity (APF, 515 nm)-forward scattered light intensity (FSC)".

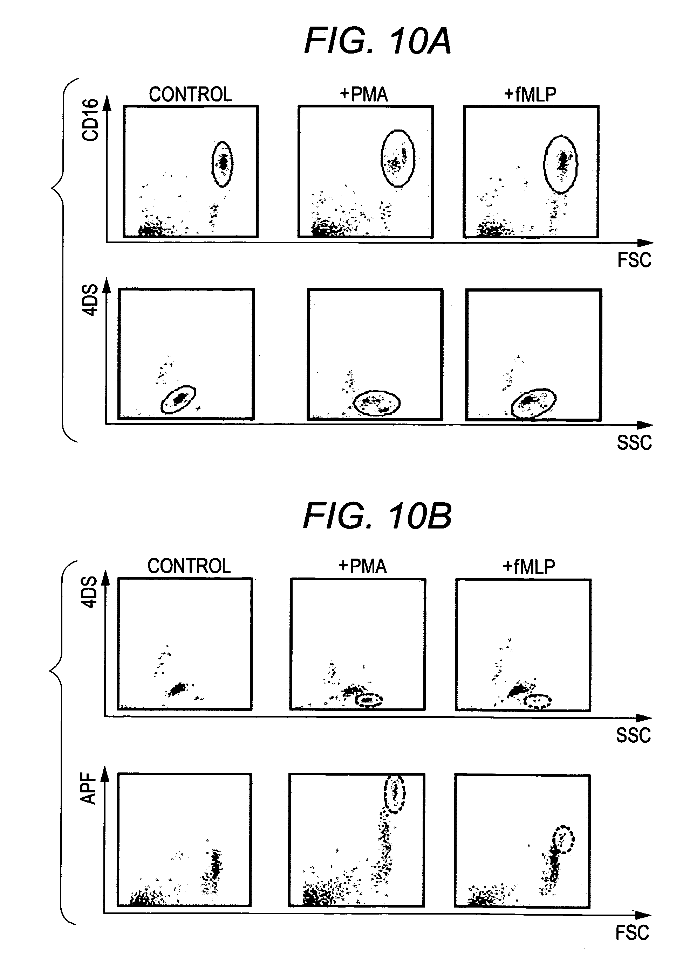

The obtained scattergrams are shown in FIGS. 10A and 10B. In FIG. 10A, a scattergram (hereinafter, referred to as "CD16-FSC") having CD16 derived fluorescence intensity and FSC as two axes and a scattergram (hereinafter, referred to as "4DS-SSC") having nucleic acid staining fluorochrome derived fluorescence intensity in Stromatolyser-4DS and SSC as two axes are shown. In FIG. 10B, a scattergram (hereinafter, referred to as "APF-FSC") having 4DS-SSC and APF derived fluorescence intensity and FSC as two axes is shown.

Particles identified as CD16 positive cells in CD16-FSC are identified as neutrophils by referring to FIG. 10A (particles surrounded by round frames in CD 16-FSC and 4DS-SSC). It is clear from 4DS-SSC that neutrophils appear on other regions in PMA and fMLP panels, in addition to the normal neutrophil appearance region shown by the control (non-stimulated) panel (particles surrounded by dotted lines in FIG. 10B). Such neutrophils appear in a lower right side (between a cluster containing neutrophils and a cluster containing eosinophils) region of the normal neutrophil appearance region. That is, the neutrophils have fluorescence intensity equal to that of the cluster containing neutrophils or less and also have scattered light intensity between that of the cluster containing neutrophils and that of the cluster containing eosinophils.

ROS production as an index of activated neutrophils of such neutrophils appearing on the lower right side of the normal neutrophil appearance region is verified based on APF-FSC. Referring to FIG. 10B, particles surrounded by dotted lines in APF-FSC are neutrophils appearing on the lower right side of the normal neutrophil appearance region in 4DS-SSC. It is clear from APF-FSC that neutrophils surrounded by dotted lines have higher ROS production than normal neutrophils.

It is clear from the above that in the scattering in 4DS-SSC, neutrophils present between the cluster containing neutrophils and the cluster containing eosinophils, that is, neutrophils having fluorescence intensity equal to that of the cluster containing neutrophils or less and also having scattered light intensity between that of the cluster containing neutrophils and that of the cluster containing eosinophils are neutrophils activated by a stimulus and whose ROS production capability is enhanced.

### (Reference Example 1)

To verify the result of Example 1 described above, whether neutrophils activated by a stimulus show an enhanced ROS production capability also in a microscope observation is examined.

A sample containing activated neutrophils is prepared from peripheral blood extracted from an able-bodied person in the same manner as in Example 1. However, only PMA is used as a stimulating substance of neutrophils. A sample (-APF) not treated by APF and a stimulating substance and a sample (+APF) not treated by a stimulating substance are prepared as control samples.

Each prepared sample is moved to a glass bottom dish coated with poly-L-lysine to observe neutrophils in the sample by confocal laser microscopes (IX81 (manufactured by Olympus Corporation), CSU-X1 (manufactured by Yokogawa Electric Corporation), and ImagEM (manufactured by Hamamatsu Photonics K. K.)).

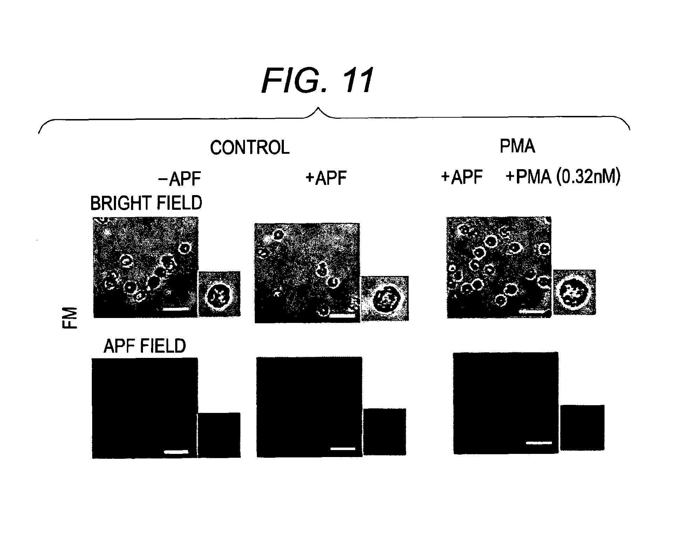

The result thereof is shown in FIG. 11. In FIG. 11, neutrophils emitting strong fluorescence derived from APF are observed in the sample to which PMA is added. Thus, it is clear that the ROS production capability of neutrophils stimulated by PMA is enhanced. Therefore, the observation result by the confocal laser microscopes agrees with the above result of Example 1.

### (Reference Example 2)

It is known that activated neutrophils show changes of form called degranulation and vacuolar degeneration as a feature thereof. Whether any cell showing changes characteristic of activated neutrophils is present in the sample prepared in Example 1 described above is examined.

A sample containing activated neutrophils is prepared from peripheral blood extracted from an able-bodied person in the same manner as in Example 1. Also, a sample not treated by a stimulating substance is prepared as a control sample.

The samples are fixed by 2.5% glutaraldehyde to prepare electron microscope samples according to the method described in the literature by the inventors (Mari Kono et al. (2009), ' Morphological definition of CD71 positive reticulocytes by various staining techniques and electron microscopy compared to reticulocytes detected by an automated hematology analyzer', Clinica Chimica Acta , Vol.404, p105-110.). The obtained samples are analyzed by using a transmission electron microscope (Hitachi H-7500).

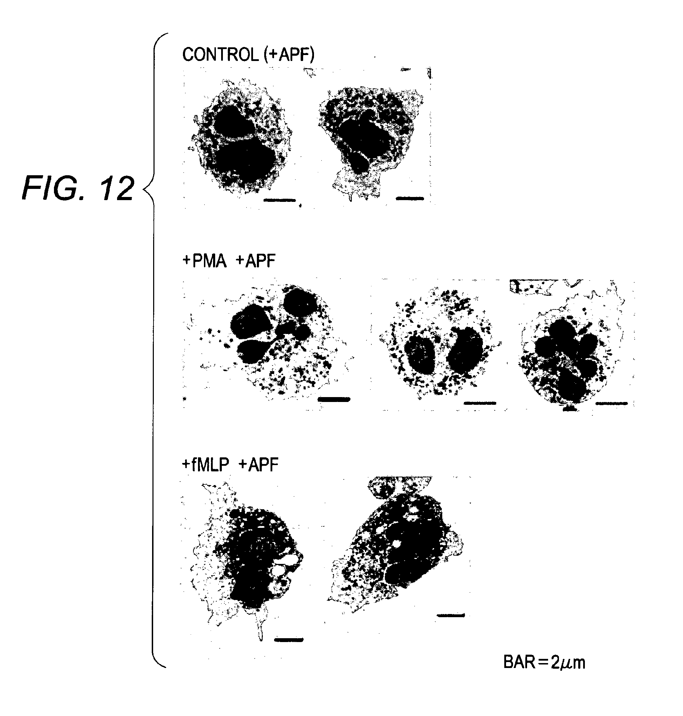

The result of observation is shown in FIG. 12. From FIG. 12, many neutrophils in which degranulation and vacuole are produced in cytoplasm are observed in samples to which PMA or fMLP is added.

From the results of Example 1, Reference Example 1, and Reference Example 2, it is clear that neutrophils in samples are surely activated by treatment using a stimulating substance. Further, measurements using a leukocyte classification reagent and flow cytometer reveal that activated neutrophils with enhanced ROS production capability in which formation of vacuole is observed in cytoplasm are present between a cluster containing neutrophils and a cluster containing eosinophils, that is, such neutrophils have fluorescence intensity equal to that of the cluster containing neutrophils or less and scattered light intensity between that of the cluster containing neutrophils and that of the cluster containing eosinophils.

### (Example 2)

Whether activated neutrophils can be detected in measurement using whole blood as the biotic sample in the same manner as in Example 1 is examined.

Peripheral blood is extracted from an able-bodied person by using a blood collection tube containing EDTA-2K for hematologic examination. PMA (manufactured by Sigma-Aldrich Co., LLC.) (final concentration: 0.32 nM) is added to the obtained blood, which is left still at room temperature for 10 min. Whole blood to which PMA is not added is used as a control sample.

The prepared sample is supplied to the automated blood cell counter XE-2100 (manufactured by Sysmex Corporation) for measurement to acquire a DIFF scattergram of 4DS-SSC. The automated blood cell counter XE-2100 automatically treats the sample prepared as described above by using Stromatolyser-4DL and Stromatolyser-4DS (manufactured by Sysmex Corporation) to prepare a measurement sample.

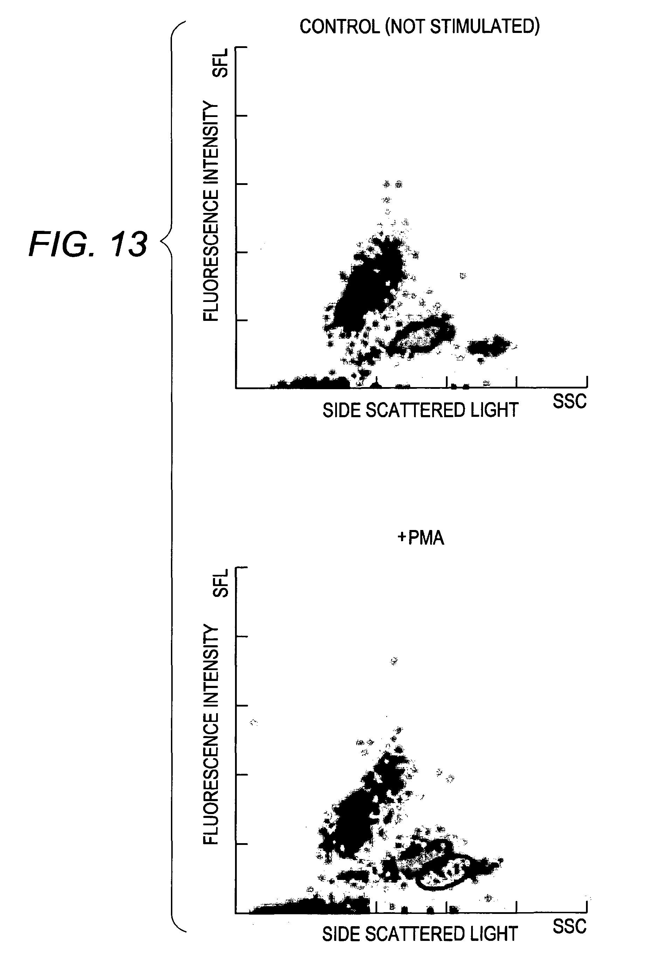

The results are shown in FIG. 13. It is clear from FIG. 13 that even if whole blood is used as a biotic sample, in a scattergram of 4DS-SSC, like in Example 1, activated neutrophils appear between a cluster containing neutrophils and a cluster containing eosinophils, that is, activated neutrophils whose fluorescence intensity is equal to that of the cluster containing neutrophils or less and whose scattered light intensity is between that of the cluster containing neutrophils and that of the cluster containing eosinophils appear (particles surrounded by a round frame). Neutrophils that appear here are considered to be activated neutrophils in which vacuoles are formed in cytoplasm by stimulus of PMA and whose ROS production capability is enhanced.

Results similar to those of Example 2 described above are obtained when measurements of the biotic sample using whole blood in Example 2 described above are performed by using an apparatus modified by incorporating a computer program for activated neutrophil detection created based on results of Example 1 into the automated blood cell counter XE-2100 (manufactured by Sysmex Corporation).

## Claims

1. A detection method for activated neutrophils, comprising:
(a) preparing a measurement sample comprising a biotic sample wherein erythrocytes are hemolyzed and nucleic acid of leukocytes is stained by a nucleic acid staining fluorochrome;
(b) irradiating the prepared measurement sample with light and measuring scattered light intensity and fluorescence intensity generated from particles in the measurement sample;
(c) classifying the leukocytes in the biotic sample into at least a cluster containing neutrophils and a cluster containing eosinophils based on the acquired scattered light intensity and fluorescence intensity; and
(d) detecting particles of which the fluorescence intensity is equal to that of the cluster containing the neutrophils or less and of which the scattered light intensity is between that of the cluster containing the neutrophils and that of the cluster containing the eosinophils as activated neutrophils.

2. The method according to claim 1, wherein the cluster containing the neutrophils further contains basophils.

3. The method according to claim 1 or 2, wherein the number of particles present between the cluster containing the neutrophils and the cluster containing the eosinophils are counted.

4. The method according to claim 3, wherein in step (d), the particles are detected as the activated neutrophils if a value counted is larger than a predetermined value, wherein the predetermined value is based on data accumulated from measurements of biotic samples containing activated neutrophils.

5. The method according to any one of claims 1 to 4, wherein erythrocytes in the measurement sample are hemolyzed by a surfactant.

6. The method according to claim 5, wherein the surfactant is at least one selected from nonionic surfactants and cationic surfactants.

7. The method according to any one of claims 1 to 6, wherein the scattered light intensity is side scattered light intensity.

## Patentansprüche

1. Nachweisverfahren für aktivierte Neutrophile, das Folgendes umfasst:
(a) Vorbereiten einer Messprobe, die eine biotische Probe umfasst, wobei Erythrozyten hämolysiert werden und Nukleinsäure von Leukozyten durch einen Nukleinsäure färbenden Fluoreszenzfarbstoff gefärbt wird;
(b) Bestrahlen der vorbereiteten Messprobe mit Licht und Messen der Streulichtintensität und der Fluoreszenzintensität, die von Partikeln in der Messprobe erzeugt werden;
(c) Klassifizieren der Leukozyten in der biotischen Probe basierend auf der gemessenen Streulichtintensität und Fluoreszenzintensität in mindestens eine Gruppe, die Neutrophile enthält, und eine Gruppe, die Eosinophile enthält; und
(d) Nachweisen von Partikeln, deren Fluoreszenzintensität jener der die Neutrophilen enthaltenden Gruppe entspricht oder geringer ist, und deren Streulichtintensität zwischen jener der die Neutrophilen enthaltenden Gruppe und jener der die Eosinophilen enthaltenden Gruppe liegt, als aktivierte Neutrophile.

2. Verfahren nach Anspruch 1, wobei die die Neutrophilen enthaltende Gruppe weiterhin Basophile enthält.

3. Verfahren nach Anspruch 1 oder 2, wobei die Anzahl der Partikel, die zwischen der die Neutrophilen enthaltenden Gruppe und der die Eosinophilen enthaltenden Gruppe vorhanden sind, gezählt wird.

4. Verfahren nach Anspruch 3, wobei in Schritt (d) die Partikel als die aktivierten Neutrophilen nachgewiesen werden, wenn ein gezählter Wert größer ist als ein vorgegebener Wert, wobei der vorgegebene Wert auf Daten basiert, die aus Messungen von biotischen Proben gesammelt wurden, die aktivierte Neutrophile enthalten.

5. Verfahren nach einem der Ansprüche 1 bis 4, wobei Erythrozyten in der Messprobe durch ein Tensid hämolysiert werden.

6. Verfahren nach Anspruch 5, wobei das Tensid mindestens eines ausgewählt aus nichtionischen und kationischen Tensiden ist.

7. Verfahren nach einem der Ansprüche 1 bis 6, wobei die Streulichtintensität Seitenstreulichtintensität ist.

## Revendications

1. Procédé de détection de neutrophiles activés, comprenant :
(a) la préparation d'un échantillon de mesure comprenant un échantillon biotique dans lequel des érythrocytes sont immobilisés et l'acide nucléique des leucocytes est coloré par un fluorochrome de coloration d'acide nucléique ;
(b) l'irradiation de l'échantillon de mesure préparé avec de la lumière et la mesure de l'intensité de lumière diffusée et de l'intensité de fluorescence générée à partir des particules dans l'échantillon de mesure ;
(c) la classification des leucocytes dans l'échantillon biotique dans au moins un groupe contenant les neutrophiles et un groupe contenant les éosinophiles sur la base de l'intensité de lumière diffusée et l'intensité de fluorescence acquises ; et
(d) la détection des particules dont l'intensité de fluorescence est égale ou inférieure à celle du groupe contenant les neutrophiles et dont l'intensité de lumière diffusée est comprise entre celle du groupe contenant les neutrophiles et celle du groupe contenant les éosinophiles en tant que neutrophiles activés.

2. Procédé selon la revendication 1, dans lequel le groupe contenant les neutrophiles contient en outre les basophiles.

3. Procédé selon la revendication 1 ou 2, dans lequel le nombre de particules présentes entre le groupe contenant les neutrophiles et le groupe contenant les éosinophiles est compté.

4. Procédé selon la revendication 3, dans lequel, dans l'étape (d), les particules sont détectées en tant que neutrophiles activés si une valeur comptée est supérieure à une valeur prédéterminée, la valeur prédéterminée étant basée sur les données accumulées à partir de mesures d'échantillons biotiques contenant des neutrophiles activés.

5. Procédé selon l'une quelconque des revendications 1 à 4, dans lequel les érythrocytes dans l'échantillon de mesure sont hémolysés par un tensioactif.

6. Procédé selon la revendication 5, dans lequel le tensioactif est au moins l'un choisi parmi des tensioactifs non ioniques et des tensioactifs cationiques.

7. Procédé selon l'une quelconque des revendications 1 à 6, dans lequel l'intensité de lumière diffusée est l'intensité de lumière diffusée latérale.
